Europäisches Patentamt

European Patent Office

Office européen des brevets

(11) Publication number: **0 351 651 B1**

# EUROPEAN PATENT SPECIFICATION

(45) Date of publication of patent specification: **15.09.93**  (51) Int. Cl.⁵: **A61K 37/26, A61K 47/14**

(21) Application number: **89112310.1**

(22) Date of filing: **06.07.89**

(54) **Insulin preparation.**

(30) Priority: **21.07.88 US 222682**

(43) Date of publication of application:
**24.01.90 Bulletin 90/04**

(45) Publication of the grant of the patent:
**15.09.93 Bulletin 93/37**

(84) Designated Contracting States:
**AT BE CH DE ES FR GB IT LI LU NL SE**

(56) References cited:
**EP-A- 0 225 189**
**FR-A- 2 313 914**
**GB-A- 1 554 157**

**H.P. Fiedler, LEXIKON DER HILFSSTOFFE FÜR
PHARMAZIE, KOSMETIK UND ANGRENZENDE
GEBIETE, 2nd ed., vol. 2, 1981, Editio Cantor,
Aulendorf (DE); pp. 832-833&NUM;**

**J. PHARM. PHARMACOL., vol. 32, 1980; E.
TOITOU et al., pp. 108-110&NUM;**

(73) Proprietor: **F. HOFFMANN-LA ROCHE AG
Postfach 3255
CH-4002 Basel(CH)**

(72) Inventor: **Campfield, Leroy Arthur
35 Depot Street
Verona, N.J. 07044(US)**
Inventor: **Davidovich, Alberto
29 Kimberly Place
Wayne, N.J. 07470(US)**
Inventor: **Infeld, Martin Howard
6 Tuers Place
Upper Montclair, N.J. 07043(US)**
Inventor: **Shah, Navnit
203 Beverly Hill Road
Clifton, N.J. 07012(US)**
Inventor: **Smith, Françoise Jeanne
55 Depot Street
Verona, N.J. 07044(US)**
Inventor: **Unowsky, Joel
203 East McClellan Avenue
Livingston, N.J. 07039(US)**

(74) Representative: **Grossner, Lutz, Dr. et al
Grenzacher Strasse 124 Postfach 3255
CH-4002 Basel (CH)**

## Description

Current pharmaceutical research has included expanded efforts to find effective means for administering insulin by non-parenteral routes such as oral, rectal and intranasal. Numerous materials have been investigated for their ability to promote the absorption of insulin delivered by non-parenteral routes of administration.

Although insulin absorption by non-parenteral means of delivery has been demonstrated in laboratory animals and diabetic patients in some cases, research on these alternative routes of administration has not led to products which have received the approval of regulatory agencies. Thus, the state of the art leaves the parenteral route as virtually the only mode of administration for diabetic patients.

EP-A 0 225 189 describes pharmaceutical compositions for oral administration that may contain surface-active agents such as a mixture of a higher fatty add salt and a fatty alcohol or glyceride as an absorption enhancer for the active ingredient. GB-A-1 554 157 describes nasal insulin preparations containing, as stabilizing agents, non-ionic surface-active agents.

Briefly described, this invention is based on the discovery that polyoxyethylene glycol (PEG)-$C_6$ to $C_{18}$ carboxylic acid glyceride esters are highly effective in promoting the absorption and bioavailability of insulin via routes of administration other than parenteral injection. Such a substance can be formulated together with insulin into various pharmaceutical dosage forms which are useful for treating conditions of hyperglycemia, such as diabetes mellitus. Suitable dosage forms comprise oral, rectal, buccal, sublingual and intranasal.

The term "polyoxyethylene glycol-$C_6$ to $C_{18}$ carboxylic acid glyceride esters" as used in connection with this invention refers to those reaction products derived from the co-reaction of polyoxyethylene glycol (or polymerizable precursor thereof, such as ethylene oxide) with a $C_6$-$C_{18}$ carboxylic acid and glycerol or with a $C_6$-$C_{18}$ carboxylic acid glyceride or glycerides. Resulting from such reactions are, typically, mixtures of a polyoxyethylene glycol-$C_6$-$C_{18}$ carboxylic acid glyceride ester (e.g., PEG-glycerol-caprate, PEG-glycerol-caprylate and PEG-glycerol-caprylate/caprate), a polyoxyethylene glycol-$C_6$ to $C_{18}$ carboxylic acid ester (e.g., PEG-caprate, PEG-caprylate and PEG-caprylate/caprate) and a glyceryl-$C_6$ to $C_{18}$ carboxylic acid ester (e.g., glyceryl mono-, di- or tricaprylate, glyceryl mono-, di- or tricaprate) as the principal components.

In greater detail, the present invention provides in one of its aspects a pharmaceutical composition comprising

(a) a blood sugar-lowering amount of insulin.

(b) an amount of a polyoxyethylene glycol-$C_6$ to $C_{18}$ carboxylic acid glyceride ester sufficient to enhance the absorption of the insulin into the body, and

(c) optionally, a pharmaceutically acceptable carrier.

The absorption enhancing agent, component (b), can be the product of an esterification reaction between a polyoxyethylene glycol, glycerol and one or more straight or branched chain $C_6$ to $C_{18}$ carboxylic acids, preferably a monofunctional acid or acids. Alternatively, component (b) may be prepared by oligomerizing or polymerizing ethylene oxide in the presence of an ester of glycerol and one or more of such $C_6$ to $C_{18}$ carboxylic acids (glyceride esters). Still another route, and the preferred one, is by co-reacting a carboxylic acid glyceride ester or esters with a fully pre-formed polyoxyethylene glycol under conditions to achieve alcoholysis.

The term "carboxylic acid glyceride ester", is employed in this description in the conventional sense to mean an ester which has been derived from glycerol and a carboxylic acid.

According to one particular procedure, involving alcoholysis, a reaction vessel is charged with stoichiometric quantities of a glyceryl-fatty acid ester or esters and polyethylene glycol, the vessel is closed and heated at atmospheric pressure to 200°., with continuous stirring commenced at 70°C., for a period of 12 to 24 hours or until the reaction is completed. The vessel is allowed to cool and the reaction product is then separated from the reaction mixture by filtration.

Examples of $C_6$ to $C_{18}$ carboxylic acids, saturated or unsaturated, which are useful for the preparations of component (b) are caproic, caprylic, capric, lauric, myristic, oleic, palmitic and stearic. Especially preferred for this invention are capric and caprylic acids, individually or together.

The polyoxyethylene glycol (PEG) used in the formation of the absorption enhancing agent is, typically, a medium to high molecular weight material, preferably having an average molecular weight in the range from 200 to 1200, and especially from 300 to 600.

A representative absorption enhancer which is suitable for purposes of this invention will most preferably have the following characteristics:

| Organoleptic Properties: | |
| --- | --- |
| Appearance: | clear oily liquid |
| Odor: | faint |
| Color: | pale yellow to yellow |

| Physical and Chemical Properties: | |
| --- | --- |
| Acid Value: | 0.2 - 0.6 |
| Sulfated ash: | less than 0.05% |
| Saponification index: | 85 - 105 |
| Iodine index: | less than 2 |
| Moisture content: | less than 0.05% |
| Free glycerin content: | approx. 2% |
| Monoglyceride content: | approx. 6 to 8% |
| Density: ($d_4^{20}$) | 1.062 - 1.068 g/cc |
| Refractive index: ($n_D^{20}$): | 1.458 - 1.462 |

Suitable absorption enhancing agents for use in this invention which are commercially available are LABRASOL®, produced by Gattefosse Corporation, Paris, France (PEG-8 caprylate/caprate glyceride esters), and SOFTIGEN®767, produced by Dynamit Nobel, West Germany (PEG-6 caprylate/caprate glyceride esters).

In providing the various dosage form compositions in accordance with this invention, the described absorption enhancing agent, component (b), is utilizable with all types of insulin effective for lowering blood sugar in the body, including but not limited to animal-source products such as crystalline forms derived from cattle (bovine) or swine (porcine) pancreatic glands (e.g., Eli Lilly's Iletin® insulins), as well as recombinant DNA-originated forms of human insulin (e.g., Lilly's Humulin®).

In addition to these more traditional types of mammalian insulin, the invention is also useful with other forms of insulin suitable for administration to humans, including various salts, derivatives and conjugates for modifying its duration of activity or other properties. Also encompassed are chemically modified derivatives of naturally occurring insulins, for example, newer molecules in which one or more amino acids have been omitted or substituted to change the properties.

In general, individual dosage forms in accordance with this invention can be composed to contain the insulin in amounts typical for the treatment of hyperglycemia, for instance, in amounts of 20, 40, 100, 200 and 500 units per dosage form.

Typically, the pharmaceutical composition of this invention contain the absorption enhancing agent, component (b), and insulin, component (a), in a weight ratio within the range from 500:1 to 1:1, and more usually from 60:1 to 20:1. For purposes of this description, each 25 units of insulin is equivalent to approximately 1 milligram of insulin.

Presently, the preferred routes of administration for this invention are by oral and buccal administration. For oral administration, the composition can be administered in the form of soft shell capsules, hard shell capsules, microcapsules, microspheres or tablets, optionally in admixture with talc or other inert ingredients, that is, pharmaceutically acceptable carriers, or in the form of aqueous solutions or suspensions, for example, in admixture with non-caloric sweetening agents, flavoring agents, stabilizers, antioxidants, preservatives, buffering agents, lubricants, colorants, thickeners and other conventional pharmaceutical additives. The capsules, spheres, tablets, solutions, etc. can be prepared in accordance with conventional methods known for these purposes.

The use of capsules is especially preferred. By way of illustration, such dosage forms are conveniently prepared by dissolving or dispersing at room temperature, with gentle mixing, a measured amount of insulin in an appropriate amount of the absorption enhancing agent, then loading into a suitable hard- or soft shell capsule (e.g., gelatin). Alternatively, if water is used, the insulin is first dissolved or suspended in water at room temperature with gentle mixing, and the insulin-water solution or suspension is added slowly to the absorption enhancing agent with mixing continued to form a uniform three-component mixture. It may be necessary to add a buffer or buffering agent to adjust the pH to within a range in which the insulin is both soluble and stable. Care should be taken not to use so much water as to dissolve or otherwise cause physical deterioration of the capsule, as will be understood by the skilled practitioner. The use of strong solvents, high temperatures or other severe conditions tending to denature the insulin should be avoided.

Examples of insulin formulations in accordance with this invention which can be prepared by the above mentioned procedures for subsequent loading into capsules are as follows.

| ORAL DOSAGE FORMULATIONS | | | |
|---|---|---|---|
| | | per capsule | per capsule |
| 1) | Insulin (crystalline) | 125 units | 250 units |
| | Absorption enhancer | 300 mg | 300 mg |
| 2) | Insulin (crystalline) | 125 units | 250 units |
| | Absorption enhancer | 300 mg | 300 mg |
| | Buffer (pH 4.0) | 10 mg | 10 mg |
| 3) | Insulin (crystalline) | 250 units | 500 units |
| | Absorption enhancer | 300 mg | 600 mg |
| | Buffer (pH 4.0) | 20 mg | 20 mg |

Preferably, the resulting capsules are provided with an enteric coating which is resistant to gastric fluids and is unaffected by them but which dissolves readily in the intestinal fluid, thereby causing insulin release.

The efficacy of particular enteric coating materials considered for use in the practice of this invention can be measured using known USP methods. By way of illustration, suitable enteric coating materials for purposes of the invention include but are not limited to the following:

## Enteric Coating Formulations

| Ingredients | % w/w |
| --- | --- |

**Solution A:**

| | |
| --- | --- |
| Hydroxypropyl methylcellulose phthalate (HPMCP) | 5.0 |
| Triacetin | 0.5 |
| Methylene chloride | 47.25 |
| Denatured alcohol | 47.25 |

**Solution B:**

| | |
| --- | --- |
| HPMCP | 10.0 |
| Titanium dioxide | 0.2 |
| Dimethyl polysiloxane | 0.05 |
| Acetone | 44.875 |
| Denatured alcohol | 44.875 |

**Solution C:**

| | |
| --- | --- |
| Cellulose acetate phthalate (CAP) | 8.5 |
| Diethyl phthalate | 1.5 |
| Titanium dioxide | 0.2 |
| Acetone | 44.9 |
| Denatured alcohol | 44.9 |

Solution D:

| | |
|---|---|
| Polyvinyl acetate phthalate | 5.0 |
| Acetylated glycerides | 0.8 |
| Methylene chloride | 47.1 |
| Denatured alcohol | 47.1 |

Solution E:

| | |
|---|---|
| Methacrylic acid or methacrylic acid ester (Eudragit®S or L, Rohm Pharma, GMBH, Wetterstadt, West Germany) | 8.0 |
| Acetone | 46.0 |
| Anhydrous alcohol | 46.0 |
| Plasticizer | q.s. |

These enteric coating materials may be applied with or without plasticizers, for example, acetylated monoglycerides, dibutyl phthalate, triethyl citrate, diethylphthalate, triacetin and dibutyl sebacate, using methods known to those skilled in the art.

The percentage of enteric coating applied is usually between about 1 and about 20 percent by weight, or more, and most desirably from about 2 to about 8 percent by weight, based on the total capsule weight.

For rectal administration, the described insulin compositions can be prepared into dosage forms suitable for the purpose, e.g., capsules or suppositories. Capsules can be prepared in a manner similar to what has been described above for the oral dosage form, but without the need for an enteric coating. Suppositories can be prepared by melting a suitable suppository base, mixing the insulin, absorption enhancer and any other ingredients into the melt, pouring the molten mixture into molds, allowing to solidify, sealing the mold and storing in well-closed containers until use. Suitable suppository bases include but are not limited to those base substances which are mixtures of mono-, di- and triglycerides of $C_{12}$ to $C_{18}$ fatty acids and are available from Dynamit Nobel Company under the trade designation WITEPSOL®. Some examples of rectal formulations are given below.

| RECTAL DOSAGE FORMULATIONS | | |
|---|---|---|
| | per suppository | per suppository |
| Insulin (crystalline) | 125 units | 500 units |
| Absorption enhancer | 415 mg | 815 mg |
| Carrier | 580 mg | 1165 mg |

For the other types of non-parenteral administration contemplated for use with this invention, suitable dosage forms can be prepared utilizing appropriate techniques well known to those skilled in the art. Such dosage forms for buccal and sublingual administration include tablets, capsules, gels, films, patches and solutions, as well as aerosol sprays for intranasal delivery. Some examples of formulations for buccal, sublingual and intranasal dosage forms are given below.

6

# EP 0 351 651 B1

## <u>BUCCAL/SUBLINGUAL DOSAGE FORMULATIONS</u>

|  | | <u>per capsule</u> | <u>per capsule</u> |
|---|---|---|---|
| 1) | Insulin (crystalline) | 125 units | 500 units |
| | Absorption enhancer | 200 mg | 400 mg |
| | Orange flavor | 5 mg | 10 mg |
| | Sodium saccharin | 1 mg | 2 mg |
| | Buffer (pH 4.0) | <u>10</u> mg | <u>20</u> mg |

|  | | <u>per capsule</u> | <u>per capsule</u> |
|---|---|---|---|
| 2) | Insulin (crystalline) | 125 units | 500 units |
| | Absorption enhancer | 200 mg | 400 mg |
| | Orange flavor | 5 mg | 10 mg |
| | Aspartame | 1 mg | 2 mg |
| | Carrier | <u>200</u> mg | <u>400</u> mg |

|  | | <u>per 0.3 ml solution</u> | <u>per 0.6 ml solution</u> |
|---|---|---|---|
| 3) | Insulin (crystalline) | 125 units | 500 units |
| | Absorption enhancer | 200 mg | 400 mg |
| | Aspartame | 1 mg | 2 mg |
| | Orange flavor | 5 mg | 10 mg |
| | Buffer (pH 4.0) q.s. | <u>0.3</u> ml | <u>0.6</u> ml |

| INTRANASAL DOSAGE FORMULATIONS | | |
|---|---|---|
| | per 0.1 ml solution | per 0.2 ml solution |
| Insulin (crystalline)<br>Absorption enhancer<br>Buffer (pH 4.0) q.s. | 125 units<br>50 mg<br><u>0.1</u> ml | 500 units<br>100 mg<br><u>0.2</u> ml |

Any of the just aforementioned dosage form compositions in accordance with this invention can also be formulated to contain pharmaceutically acceptable adjuvants typical for the particular dosage form involved, including but not limited to stabilizers, flavoring agents, antioxidants preservatives, buffering agents, colorants, bulking agents and lubricants, which may be selected from among materials known for such purposes and added in conventional amounts.

The usefulness of compositions according to this invention for the treatment of hyperglycemia is demonstrated in two separate in vivo animal models (rats and dogs), as follows.

7

IN VIVO (RATS)

Female Wistar rats and Sprague-Dawley rats, having a body weight of from 210 to 260 grams, were deprived of food for 1 to 2 hours prior to surgery. The animals were anesthetized with sodium pentobarbital, using 50 mg of the anesthetic per kilogram of animal body weight, and cardiac and duodenal cannulas were then implanted. Before surgery, the cardiac cannulas were filled and mounted with 1 c.c. syringes containing heparin saline solution at a concentration of 50 units per milliliter. The cardiac cannulas were implanted in the rats through the jugular vein at or about 34 mm toward the atria (For additional details on the procedure, see Smith and Campfield, American Journal of Physiology, 251: R70-76, 1986).

The duodenal cannulas were made of 0.86 mm (inner diameter) polyethylene tubing having a length of 150 mm and mounted with a 1 c.c. syringe filled with 0.9% saline solution. Implantation of the duodenal cannulas was made through an incision in the duodenal wall 10 mm down from the pyloric valve. In each case, approximately 15 mm of the tubing was inserted through the incision and tied with sutures around the incision.

All incisions were closed with sutures. Following completion of the surgery, 200 units of heparin (0.2 ml of 1000 units/ml, followed by 0.2 ml of isotonic saline) were injected into the cardiac cannula. The experimental protocol was commenced 60 minutes later.

Insulin Administration

Insulin (Eli Lilly's Iletin® U500, 500 units per milliliter) was gently mixed with varying concentrations of polyoxyethylene glycol (8) caprylate/caprate glyceride esters (Gattefosse's Labrasol®) prior to infusion. Particular concentrations of these materials in each incidence of administration are detailed further below and shown in the accompanying graphs. For each test animal, a 0.5 ml dose of solution was infused into the exteriorized portion of the implanted duodenal cannula, using a 1 c.c. tuberculin syringe attached to a 20-gauge needle.

Continuous Blood Glucose Recording

A YSI Model 23A Glucose Analyzer (Yellow Springs Instrument Co., Inc.) and glucose-oxidase method were used to continuously measure the blood glucose concentation, in units of mg/dl, in rats. (For details, see Campfield et al., Brain Research Bulletin 14: 605-615 (1985)).

Following at least 10 minutes of stable blood glucose monitoring (± 1%), administration of insulin-absorption enhancer mixture was begun and blood glucose recording was continued for up to 60 minutes. Upon termination of experiments in each test rat, the correct placement of the duodenal cannula was verified by administration of 1 ml of trypan blue. This allowed verification of proper intestinal delivery of the mixture. Changes in the level of blood glucose were calculated as a function of time and magnitude over the baseline. At least three runs were performed, each on a different test rat. If the infusion of insulin and absorption enhancer significantly reduced the blood glucose level (i.e., greater than 10%), a dose-response curve of the insulin-absorption enhancer mixture was further evaluated in discrete sampling experiments, the procedure of which is described below.

Discrete Sampling

Baseline blood samples of 0.25 ml volume were collected from the test rat at 15 minutes prior to administration of the insulin-absorption enhancer mixture. Blood samples were also collected at 2, 15, 30, 45, 60 and 90 minute-intervals following insulin-absorption enhancer administration. Blood volume was replaced after withdrawl of each sample with 0.25 ml of isotonic saline. Withdrawn blood samples were immediately spun using a micro-centrifuge (Eppendorf Co., Model No. 5415) for 2 minutes at 14,000 r.p.m. Plasma was collected into micro-beakers and stored at -20°C for subsequent determination of glucose and insulin concentrations, using the assay procedures described below.

Plasma Glucose and Insulin Assays

The plasma glucose concentrations of the test rats over time were measured in 25-microliter samples using a YSI Model 23A Glucose Analyzer, Yellow Springs Instrument Co., Inc., which employs a glucose-oxidase method (see reference above for details). The results were expressed in mg/dl and percent change of baseline. Data were averaged across test groups and expressed as mean ± S.E. M.

The plasma insulin concentrations of the test rats were measured in 25-microliter samples using a competitive protein binding microassay, with pork insulin as the standard (see Campfield et al., American Journal of Physiology, 244: R629-634, 1983). The mimimum detectable insulin concentration was 0.04 ng/ml and the maximum readable concentration (undiluted) was 10 ng/ml. The results were expressed in ng/ml, and the data were averaged and expressed as mean ± S.E.M.

Dose-Response Studies of the Absorption of 20 Units of Insulin as a Function of Absorption Enhancer Concentration - Single Dose (Rats)

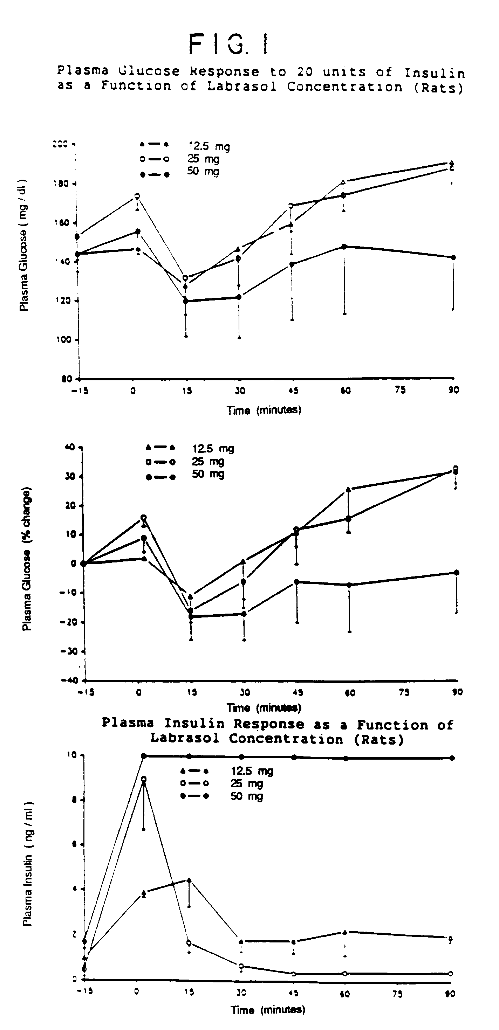

Three different concentrations of polyoxyethylene glycol (8) caprylate/caprate glyceride esters (Gattefoss's Labrosol®) as the absorption enhancer were mixed with a constant dose of 20 units of porcine insulin (Lilly's Illetin® U500) and infused intraduodenally into anesthetized rats using the method referred to above, such that the rats received 12.5, 25 or 50 mg of the absorption enhancer, which corresponds to approximately a 2.5, 5 or 10% solution, respectively, of absorption enhancer. The effect of these infusions on the plasma glucose concentration, expressed both as an absolute and a percent change from the baseline, as well as the plasma insulin concentrations as a function of time are shown in Figure 1. The data points represent mean ± S.E.M.

As is shown, intraduodenal administration of mixtures of the absorption enhancer and insulin resulted in a decrease in the plasma glucose of about 10-20% after 15 minutes. The plasma glucose returned to the baseline after 45 minutes in the 12.5 and 25 mg groups, while remaining slightly suppressed in the 50 mg group. Plasma insulin concentrations rose rapidly from baseline levels to a peak after only 2 minutes following infusion in all three groups (12.5, 25 and 50 mg). Plasma insulin then returned to the baseline in the 12.5 and 25 mg groups after 30 minutes, while remaining elevated during the entire 90-minute post-infusion period in the 50 mg group.

Dose-Response Studies of the Absorption of Insulin in the Presence of 25 mg of Absorption Enhancer as a Function of Insulin Concentration - Single Dose (Rats)

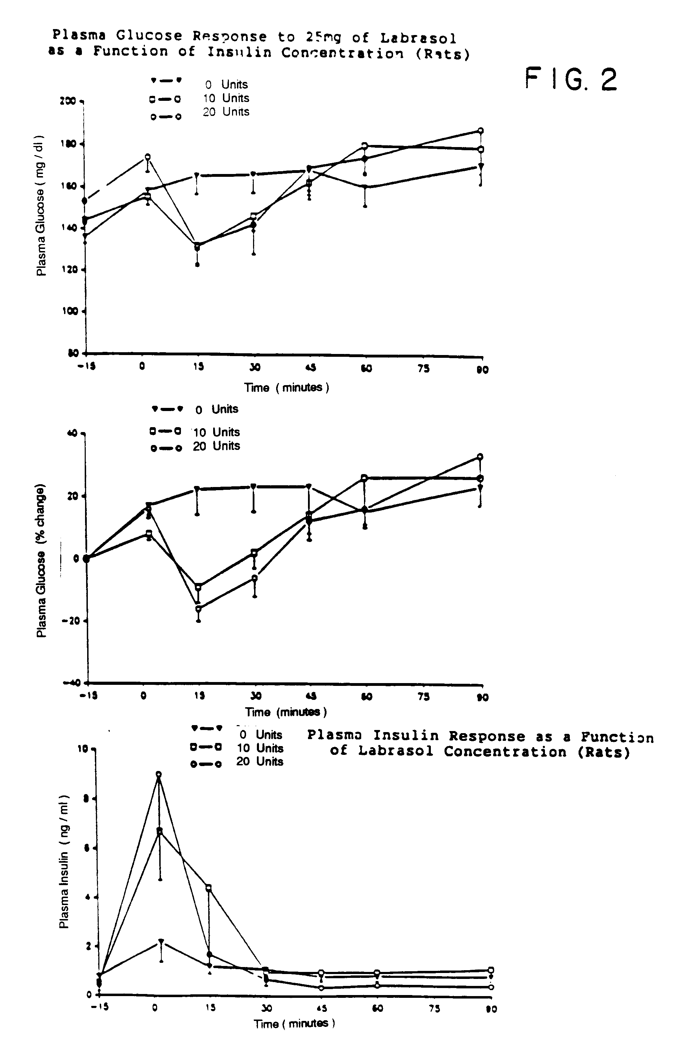

Three different doses of porcine insulin (Lilly's Iletin® U500), 0, 10 or 20 units, were mixed with a constant dose of 25 mg (5% solution) absorption enhancer (Gattefoss's Labrosol®) and the mixture were infused introduodenally into anesthetized rats using the method referred to above. The effects on plasma glucose and insulin levels over time are shown in Figure 2. The data points represent mean ± S.E.M.

Intraduodenal administration of the absorption enhancer-insulin mixture at these doses resulted in a decrease in plasma glucose of about 10-20% 15 minutes after infusion, as shown. The plasma glucose returned to the baseline level after 45 minutes. Administration of the absorption enhancer alone was without effect on the plasma glucose level. The plasma insulin concentration increased rapidly from the baseline level to a peak in both groups (10 and 20 units ) 2 minutes after infusion, then returned to the baseline level by 30 minutes after infusion.

Dose-Response of the Absorption of Insulin in the Presence of 50 mg of Absorption Enhancer as a Function of Insulin Concentration - Single Dose (Rats)

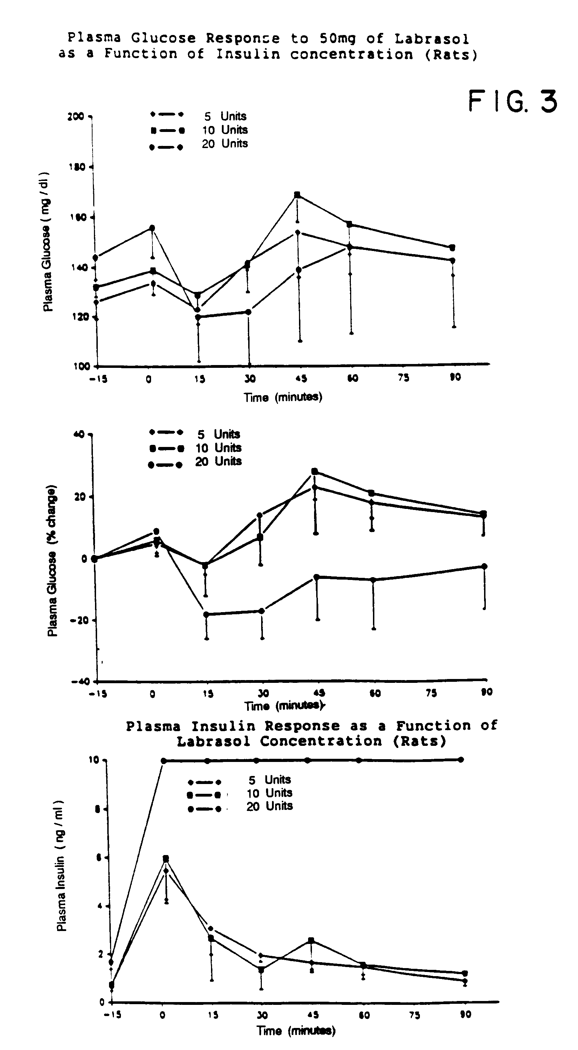

Three different doses of porcine insulin (Lilly's Iletin® U500), 5, 10 or 20 units, were infused intraduodenally into anesthetized rats using the method referred to above. The effects are shown in Figure 3, with the data points given in mean ± S.E.M.

Intraduodenal administration of absorption enhancer and insulin at these doses also resulted in a dose-dependent decrease in the plasma glucose level within 15 minutes after infusion. While the decrease in plasma glucose was relatively small in the 5-unit and 10-unit groups, the plasma glucose level had risen by an average of 22% by this time in the control group (25 mg of absorption enhancer alone - see Figure 2).

Plasma insulin concentrations increased rapidly from baseline levels to a peak 2 minutes after infusion in all three groups and remained elevated during the entire 90-minute post-infusion period in the 20-unit test group. These data demonstrate a dose-dependent absorption of insulin in the presence of 50 mg of the absorption enhancer.

Comparison of 25 mg and 50 mg Absorption Enhancer Concentrations of Insulin Absorption

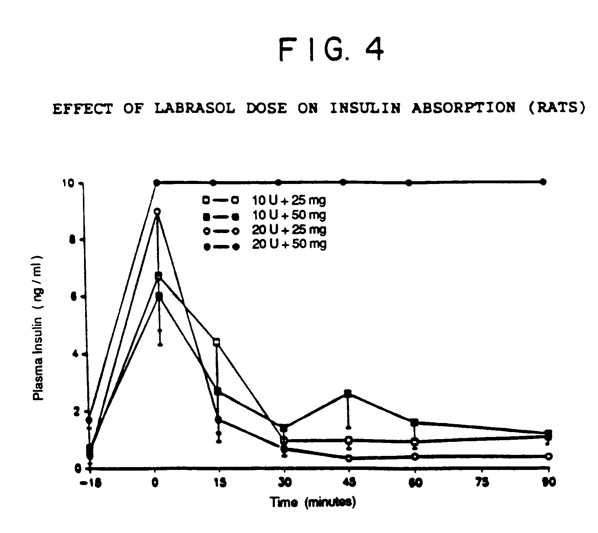

Comparison of the plasma insulin responses for the 10-and 20-unit insulin groups with these concentrations of absorption enhancer shows that while additional amounts of absorption enhancer did not increase the absorption of insulin from the 10-unit mixture, it did result in a significant enhancement of insulin absorption from the 20-unit mixture. These results are depicted in Figure 4.

In the foregoing experiments, the intestinal absorption of insulin in the presence of the absorption enhancer is shown to be large, reproducible and dose-dependent.

## Multiple Dose Insulin/Absorption Enhancer (Rats)

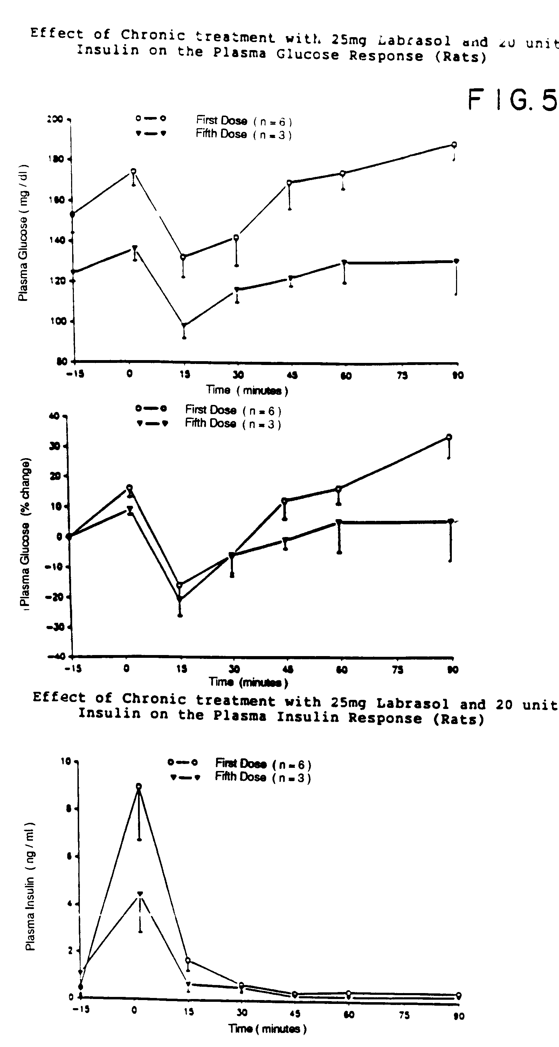

Three rats with chronic intraduodenal cannulas received four daily doses of 25 mg of Labrasol® and 20 units of porcine insulin. On the next day, the rats were anesthetized and the plasma glucose and insulin responses to the fifth dose were measured. The glucose and insulin responses following the fifth dose are compared with the responses to a single dose in naive rats of the same age and sex in Figure 5. Data are mean ± S.E.M.

Plasma glucose concentrations decreased to a nadir (-21%) after 15 minutes and returned to baseline levels at 45 minutes in both multiple dose-treated and naive rats. Plasma insulin concentration rose rapidly to a large peak (4-9 fold) at 2 minutes post-infusion and returned to baseline at 30 minutes in both multiple dose treatment and single dose treatment groups. These studies demonstrate that the ability of 25 mg of Labrasol® to enhance the intestinal absorption of insulin is retained for at least 5 days in the animals tested.

## IN VIVO (DOGS)

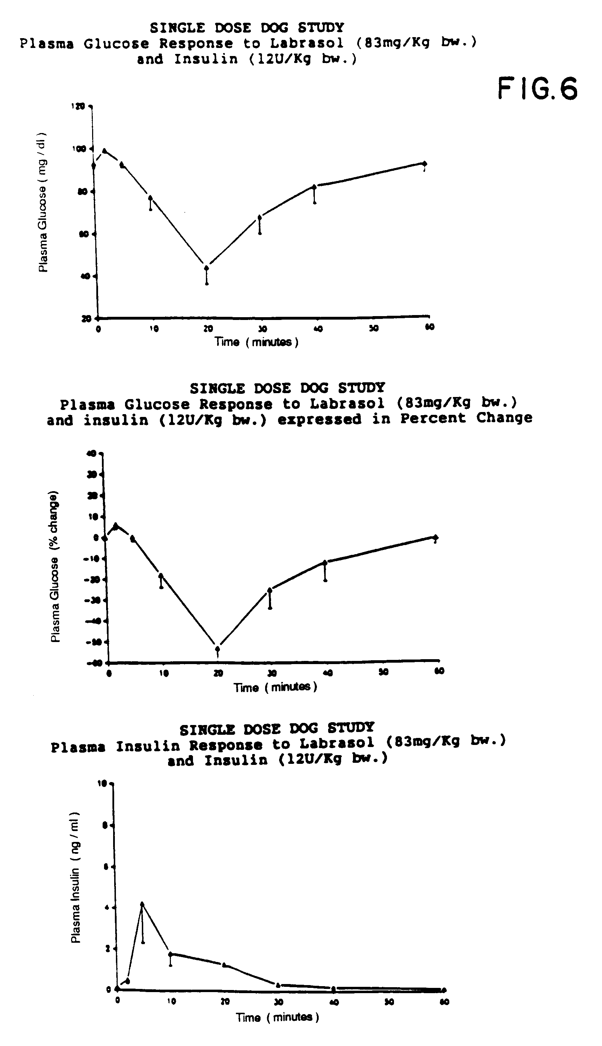

Mixtures of polyoxyethylene glycol (8) caprylate/caprate glyceride esters (Gattefosse's Labrasol®) and insulin (Lilly's Iletin® U500) were infused intraduodenally into five fasting, awake beagle dogs chronically fitted with a modified Thomas cannula implanted in the distal duodenum. Each dog weight about 10-15 kilograms. In each case, the dosage amount of the absorption enhancer was approximately 83 milligrams per kilogram of body weight and of the insulin was approximately 12 units per kilogram of body weight. Blood samples for monitoring glucose and insulin levels were obtained predose and 2, 5, 10, 20, 30, 40 and 60 minutes post dose. The results are shown in Figure 6 for both plasma glucose and plasma insulin concentrations, with data points representing mean ± S.E.M.

Intraduodenal administration of the absorption enhancer-insulin mixture resulted in a significant decrease in plasma glucose (-53%) by 20 minutes after infusion. Plasma insulin concentration increased within 2 minutes, rose to a peak at 5 minutes, and remained elevated after 20 minutes. These studies show that the absorption enhancer does increase the intestinal absorption of insulin in conscious, unrestrained dogs to produce a significant, reproducible augmentation of plasma insulin.

The administration of absorption-enhanced insulin-containing dosage forms according to this invention and the resulting effect on plasma glucose and plasma insulin concentrations are shown in the following animal studies.

## Buccal Administration

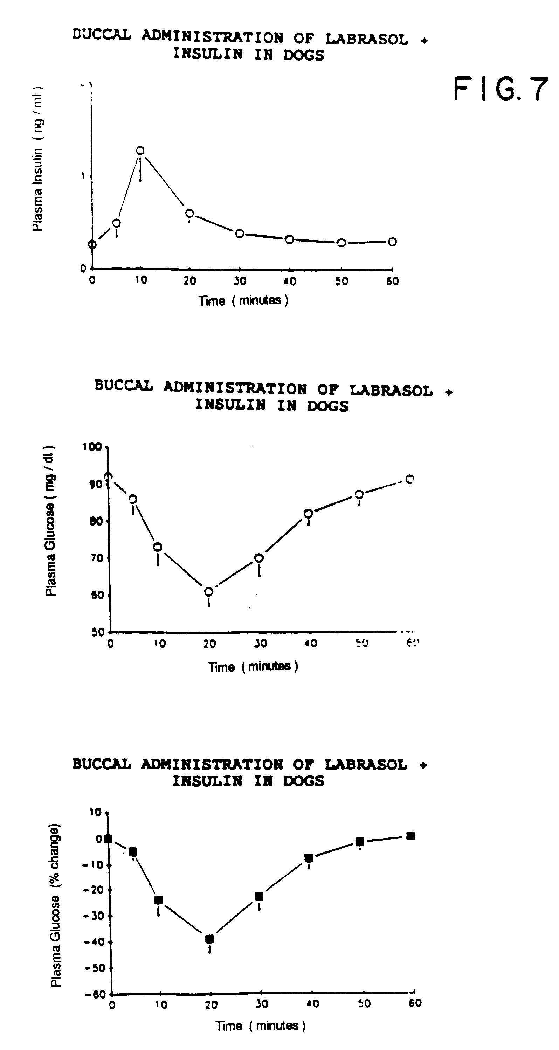

A mixture of Labrasol® and insulin was placed in the cheek pouch of each of ten beagle dogs (weighing approximately 12 kg each). The formulation was prepared by mixing 200 mg (0.2 ml) of Labrasol® with 120 units of crystalline insulin (Sigma Chemical Company) dissolved in 0.1 ml of citric acid solution, 1% w/v (pH 3.2), resulting in a final volume of 0.3 ml, which was administered to each dog. The dosage amount of the absorption enhancer was 16.7 mg per kilogram of body weight and of the insulin was 10 units per kilogram of body weight. The results are shown in Figure 7 for plasma glucose and plasma insulin concentrations, with data points representing mean ± S.E.M.

Buccal administration of the absorption enhancer-insulin mixture resulted in a significant decrease in plasma glucose (-39%) by 20 minutes after dosing. Plasma insulin concentration increased to a 5-fold peak after 10 minutes and remained elevated for 40 minutes after dosing. This study shows that significant absorption of insulin into the bloodstream occurs following buccal administration of the absorption enhancer-insulin mixture.

## Oral Administration of Enteric Coated Capsules

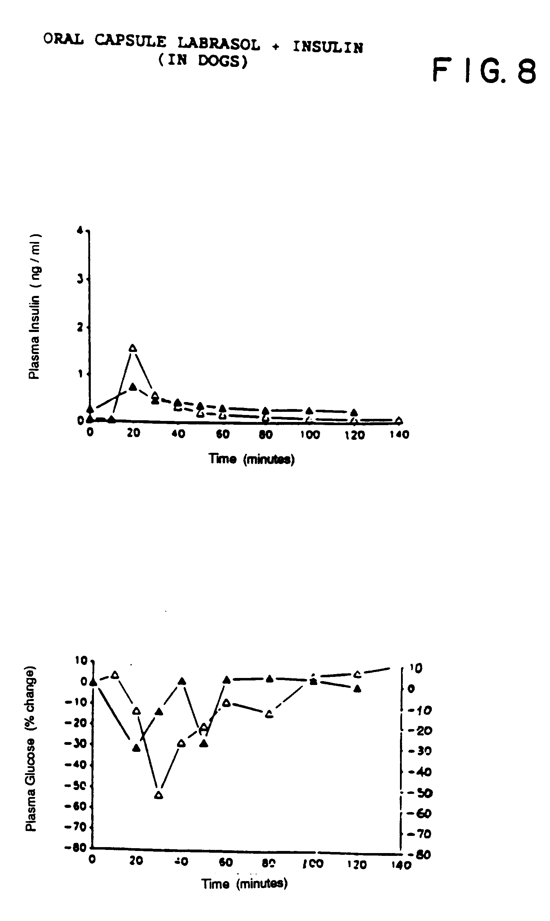

Enteric coated capsules containing 450 mg of Labrasol® 60 units of crystalline insulin (Sigma Chemical Company) and 10 mg of water were prepared. Each capsule contained enough absorption enhancer-insulin formulation for a 5 kg dog. Two capsules were administered to each of ten awake beagle dogs weighing approximately 10-15 kg (that is, about 90 mg of absorption enhancer and 12 units of insulin per kilogram of body weight, consistent with the intraduodenal dog studies). In two separate experiment, the results from a representative dog are shown in Figure 8 for both plasma glucose and plasma insulin concentrations.

Plasma glucose decreased in both experiments (-53% at 30 minutes and -32% at 20-50 minutes) and returned to baseline by 100 minutes after dosing. Plasma insulin increased to a peak at 20 minutes and remained elevated for 60 minutes. These studies, together with similar studies in other dogs, demonstrate a significant absorption of insulin into the bloodstream following oral dosing with enteric coated capsules containing the absorption enhancer-insulin formulation.

The described dosage forms of this invention are, as mentioned and as demonstrated in the foregoing studies, useful to treat hyperglycemia. Such a method of treatment constitutes another aspect of this invention.

In the practice of the invention, the dose of the described insulin composition to be administration and the frequency of administration will depend on the potency and duration of activity of the insulin and the degree of absorption conferred by the absorption enhancer, as well as the severity of the condition, presence of neutralizing antibodies, degree of insulin resistance, duration of diabetes, age of the subject being treated and other factors known to the skilled practitioner. Doses of insulin contemplated for use in the practice of the invention are typical for insulin treatment as delivered to the bloodstream by injectable forms of insulin, for example, from 25 to 500 units per day, depending on specific needs, either as a single dose or in divided doses per day.

## BRIEF DESCRIPTION OF THE DRAWINGS

Fig. 1 - This drawing graphs plasma glucose and plasma insulin responses versus time as a function of absorption enhancer concentration at a constant dose of insulin (20 units), using rats as the subjects.

Fig. 2 - This drawing graphs plasma glucose and plasma insulin responses versus time as a function of insulin concentration at a constant amount of absorption enhancer (25 mg), using rats as the subjects.

Fig. 3 - This drawing graphs plasma glucose and plasma insulin responses versus time as a function of insulin concentration at a constant amount of absorption enhancer (50 mg), using rats as the subjects.

Fig. 4 - This drawing graphically compares the effect of absorption enhancer concentration on insulin absorption assessed by plasma insulin concentration in rats.

Fig. 5 - This drawing graphs plasma glucose and plasma insulin responses, using a constant dose of insulin and absorption enhancer, in a multiple dose study in rats.

Fig. 6 - This drawing graphs plasma glucose and plasma insulin responses versus time following the intraduodenal administration of a single dose of an insulin-absorption enhancer mixture to dogs.

Fig. 7 - This drawing graphs plasma glucose and plasma insulin responses versus time following the buccal administration of an insulin-absorption enhancer mixture to dogs.

Fig. 8 - This drawing graphs plasma glucose and plasma insulin responses versus time following the oral administration of an insulin-absorption enhancer mixture to dogs.

**Claims**

**Claims for the following Contracting States : AT, BE, CH, DE, FR, GB, IT, LI, LU, NL, SE**

**1.** A non-parenteral pharmaceutical compositions comprising
   (a) a blood sugar-lowering amount of insulin.
   (b) an amount of a polyoxyethylene glycol-$C_6$ to $C_{18}$ carboxylic acid glyceride ester sufficient to enhance the absorption of the insulin into the body, and
   (c) optionally, a pharmaceutically acceptable carrier.

**2.** A composition according to claim 1, in which the $C_6$ to $C_{18}$ carboxylic acid is a fatty acid or combination of fatty acids.

**3.** A composition according to claim 2, in which the fatty acid is saturated.

**4.** A composition according to claim 3, in which the saturated fatty acid is capric acid or caprylic acid or a mixture of both.

**5.** A composition according to claim 1, in which the polyoxyethylene glycol has an average molecular weight in the range from 200 to 1200.

**6.** A composition according to claim 1, in which the weight ratio of component (b) to component (a) is in the range from 500:1 to 1:1.

**7.** A composition according to claim 6, in which the weight ratio of component (b) to component (a) is in the range from 60:1 to 20:1.

**8.** A composition according to claim 1, which contains from 20 to 500 units of insulin.

**Claims for the following Contracting State : ES**

**1.** Process for the preparation of a non-parenteral pharmaceutical compositions comprising
(a) a blood sugar-lowering amount of insulin.
(b) an amount of a polyoxyethylene glycol-$C_6$ to $C_{18}$ carboxylic add glyceride ester sufficient to enhance the absorption of the insulin into the body, and
(c) optionally, a pharmaceutically acceptable carrier.
characterized in that an amount of insulin is dissolved or dispersed in an appropriate amount of the absorption enhancer whereupon the mixture is brought into a non-parenteral application formulation.

**2.** A process according to Claim 1, in which the $C_6$ to $C_{18}$ carboxylic add is a fatty add or combination of fatty acids.

**3.** A process according to Claim 2, in which the fatty add is saturated.

**4.** A process according to Claim 3, in which the saturated fatty add is capric add or caprylic acid or a mixture of both.

**5.** A process according to Claim 1, in which the polyoxyethylene glycol has an average molecular weight in the range from 200 to 1200.

**6.** A process according to Claim 1, in which the weight ratio of component (b) to component (a) is in the range from 500 : 1 to 1 : 1.

**7.** A process according to Claim 6, in which the weight ratio of component (b) to component (a) is in the range from 60 : 1 to 20 : 1.

**8.** A process according to Claim 1 for the preparation of compositions containing from 20 to 500 units of insulin.

**Patentansprüche**

**Patentansprüche für folgende Vertragsstaaten : AT, BE, CH, DE, FR, GB, IT, LI, LU, NL, SE**

**1.** Eine nicht-parenterale pharmazeutische Komposition, umfassend
(a) eine Blutzucker-senkende Menge Insulin,
(b) eine für die Verstärkung der Resorption des Insulins hinreichende Menge Polyoxyäthylenglykol-$C_6$ - $C_{18}$-carbonsäureglyzeridester, und
(c) gewünschtenfalls einen pharmazeutisch annehmbaren Träger.

**2.** Eine Komposition nach Anspruch 1, in der die $C_6$ - $C_{18}$ - Carbonsäure eine Fettsäure oder eine Kombination von Fettsäuren ist.

**3.** Eine Komposition nach Anspruch 2, in der die Fettsäure gesättigt ist.

**4.** Eine Komposition nach Anspruch 3, in der die gesättigte Fettsäure Caprinsäure oder Caprylsäure oder ein Gemisch der beiden ist.

**5.** Eine Komposition nach Anspruch 1, in der das Polyoxyäthylenglykol ein mittleres Molekulargewicht im Bereich von 200 bis 1200 hat.

12

**6.** Eine Komposition nach Anspruch 1, in der das Gewichtsverhältnis von Komponente (b) zu Komponente (a) im Bereich von 500:1 bis 1:1 ist.

**7.** Eine Komposition nach Anspruch 6, in der das Gewichtsverhältnis von Komponente (b) zu Komponente (a) im Bereich von 60:1 bis 20:1 ist.

**8.** Eine Komposition nach Anspruch 1, die 20 bis 500 Einheiten Insulin enthält.

**Patentansprüche für folgenden Vertragsstaat : ES**

**1.** Verfahren zur Herstellung einer nicht-parenteralen pharmazeutischen Komposition, umfassend
(a) eine Blutzucker-senkende Menge Insulin,
(b) eine für die Verstärkung der Resorption des Insulins hinreichende Menge Polyoxyäthylenglykol-$C_6$ - $C_{18}$-carbonsäureglyzeridester, und
(c) gewünschtenfalls einen pharmazeutisch annehmbaren Träger,
dadurch gekennzeichnet, dass eine Insulinmenge in einer passenden Menge Resorptionsverstärker gelöst oder dispergiert wird, worauf das Gemisch in eine nicht-parenterale Anwendungsform gebracht wird.

**2.** Verfahren nach Anspruch 1, in dem die $C_6$ - $C_{18}$ - Carbonsäure eine Fettsäure oder eine Kombination von Fettsäuren ist.

**3.** Verfahren nach Anspruch 2, in dem die Fettsäure gesättigt ist.

**4.** Verfahren nach Anspruch 3, in dem die gesättigte Fettsäure Caprinsäure oder Caprylsäure oder ein Gemisch der beiden ist.

**5.** Verfahren nach Anspruch 1, in dem das Polyoxyäthylenglykol ein mittleres Molekulargewicht im Bereich von 200 bis 1200 hat.

**6.** Verfahren nach Anspruch 1, in dem das Gewichtsverhältnis von Komponente (b) zu Komponente (a) im Bereich von 500:1 bis 1:1 ist.

**7.** Verfahren nach Anspruch 6, in dem das Gewichtsverhältnis von Komponente (b) zu Komponente (a) im Bereich von 60:1 bis 20:1 ist.

**8.** Verfahren nach Anspruch 1 zur Herstellung von Kompositionen, die 20 bis 500 Einheiten Insulin enthalten.

**Revendications**
**Revendications pour les Etats contractants suivants : AT, BE, CH, DE, FR, GB, IT, LI, LU, NL, SE**

**1.** Composition pharmaceutique non-parentérale comprenant
(a) une quantité d'insuline abaissant le sucre sanguin,
(b) une quantité d'un polyoxyéthylène glycol-ester glycéridique d'acide carboxylique en $C_6$ à $C_{18}$ suffisante pour accroître l'absorption du l'insuline dans le corps, et
(c) facultativement, un support pharmaceutiquement acceptable.

**2.** Composition selon la revendication 1, dans laquelle l'acide carboxylique an $C_6$ à $C_{18}$ est un acide gras ou une combinaison d'acides gras.

**3.** Composition selon la revendication 2, dans laquelle l'acide gras est saturé.

**4.** Composition selon la revendication 3, dans laquelle l'acide gras saturé est l'acide caprique ou l'acide caprylique, ou un mélange des deux.

**5.** Composition selon la revendication 1, dans laquelle le polyoxyéthylène glycol à un poids moléculaire moyen situé dans un intervalle allant de 200 à 1200.

6. Composition selon la revendication 1, dans laquelle le rapport pondéral du composant (b) au composant (a) est dans un intervalle allant de 500:1 à 1:1.

7. Composition selon la revendication 6, dans laquelle la rapport pondéral du composant (b) au composant (a) est dans un intervalle allant de 60:1 à 20:1.

8. Composition selon la revendication 1, qui contient de 20 à 500 unités d'insuline.

**Revendications pour l'Etat contractant suivant : ES**

1. Procédé de préparation d'une composition pharmaceutique non-parentérale comprenant
   (a) une quantité d'insuline abaissant le sucre sanguin,
   (b) une quantité d'un polyoxyéthylène glycol-ester glycéridique d'acide carboxylique en $C_6$ à $c_{18}$ suffisante pour accroître l'absorption de l'insuline dans le corps, et
   (c) facultativement, un support pharmaceutiquement acceptable,
   caractérisé en ce qu'on dissout ou disperse une certaine quantité d'insuline dans une quantité appropriée d'améliorateur d'absorption, après quoi on introduit le mélange dans une formulation d'application non-parentérale.

2. Procédé selon la revendication 1, dans lequel l'acide carboxylique en $C_6$ à $C_{18}$ est un acide gras ou une combinaison d'acides gras.

3. Procédé selon la revendication 2, dans lequel l'acide gras est saturé.

4. Procédé selon la revendication 3, dans lequel l'acide gras saturé est l'acide caprique ou l'acide caprylique, ou un mélange des deux.

5. Procédé selon la revendication 1, dans lequel le polyoxyéthylène glycol a un poids moléculaire moyen situé dans un intervalle allant de 200 à 1200.

6. Procédé selon la revendication 1, dans lequel le rapport pondéral du composant (b) au composant (a) est dans un intervalle allant de 500:1 à 1:1.

7. Procédé selon la revendication 6, dans lequel le rapport pondéral du composant (b) au composant (a) est dans un intervalle allant de 60:1 à 20:1.

8. Procédé selon la revendication 1 de préparation de compositions contenant de 20 à 500 unités d'insuline.

EP 0 351 651 B1

# FIG. 1

**Plasma Glucose Response to 20 units of Insulin as a Function of Labrasol Concentration (Rats)**

**Plasma Insulin Response as a Function of Labrasol Concentration (Rats)**

Plasma Glucose Response to 25mg of Labrasol
as a Function of Insulin Concentration (Rats)

FIG. 2

▼—▼  0 Units
□—□  10 Units
○—○  20 Units

▼—▼  0 Units
□—□  10 Units
○—○  20 Units

▼—▼  0 Units
□—□  10 Units   Plasma Insulin Response as a Function
○—○  20 Units      of Labrasol Concentration (Rats)

16

Plasma Glucose Response to 50mg of Labrasol
as a Function of Insulin concentration (Rats)

FIG. 3

Plasma Insulin Response as a Function of
Labrasol Concentration (Rats)

# F I G. 4

EFFECT OF LABRASOL DOSE ON INSULIN ABSORPTION (RATS)

Effect of Chronic treatment with 25mg Labrasol and 20 units
Insulin on the Plasma Glucose Response (Rats)

# F I G. 5

Effect of Chronic treatment with 25mg Labrasol and 20 units
Insulin on the Plasma Insulin Response (Rats)

19

**SINGLE DOSE DOG STUDY**
Plasma Glucose Response to Labrasol (83mg/Kg bw.)
and Insulin (12U/Kg bw.)

# FIG.6

**SINGLE DOSE DOG STUDY**
Plasma Glucose Response to Labrasol (83mg/Kg bw.)
and insulin (12U/Kg bw.) expressed in Percent Change

**SINGLE DOSE DOG STUDY**
Plasma Insulin Response to Labrasol (83mg/Kg bw.)
and Insulin (12U/Kg bw.)

BUCCAL ADMINISTRATION OF LABRASOL +
INSULIN IN DOGS

FIG.7

BUCCAL ADMINISTRATION OF LABRASOL +
INSULIN IN DOGS

BUCCAL ADMINISTRATION OF LABRASOL +
INSULIN IN DOGS

EP 0 351 651 B1

ORAL CAPSULE LABRASOL + INSULIN
(IN DOGS)

F I G. 8

22